# EUROPÄISCHE PATENTSCHRIFT

(11) **EP 0 749 955 B1**
(45) Veröffentlichungstag und Bekanntmachung des Hinweises auf die Patenterteilung: **16.08.2000**
(21) Anmeldenummer: 96109317.6
(22) Anmeldetag: 11.06.1996
(51) Int. Cl.: C07C 68/08, C07C 69/96

(54) **Verfahren zur Herstellung von Diarylcarbonaten**
Process for preparing diaryl carbonates
Procédé pour la préparation des carbonates de diaryles

(30) Priorität: 23.06.1995 DE 19523390
(43) Veröffentlichungstag der Anmeldung: 27.12.1996
(73) Patentinhaber: BAYER AG, 51368 Leverkusen (DE)
(72) Erfinder: Buysch, Hans-Josef, Dr., 47809 Krefeld (DE); Hesse, Carsten, Dr., 47800 Krefeld (DE); Rechner, Johann, Dr., 47906 Kempen (DE)

(56) Entgegenhaltungen:
- EP-A- 0 413 215
- EP-A- 0 450 442
- EP-A- 0 460 732
- DE-A- 2 738 437
- DE-A- 4 403 075

## Beschreibung

Die vorliegende Erfindung betrifft ein Verfahren zur Herstellung von Diarylcarbonaten durch Umsetzung einer aromatischen Hydroxyverbindung (z.B. Phenol) mit Kohlenmonoxid und Sauerstoff in Gegenwart eines Katalysators, eines Cokatalysators, eines quartären Salzes und einer Base, das dadurch gekennzeichnet ist, daß man das Reaktionswasser kontinuierlich entfernt, indem man ständig einen Teilstrom der Reaktionslösung aus dem Reaktor entnimmt, unter weitgehend isothermen Bedingungen das Wasser bei reduziertem Druck abzieht und die Reaktionslösung anschließend zurückführt.

Es ist bekannt, organische Carbonate durch oxidative Umsetzung einer aromatischen Hydroxyverbindung mit Kohlenmonoxid in Gegenwart eines Edelmetall-Katalysators und herzustellen (DE-OS 27 38 437). Als Edelmetall wird bevorzugt Palladium eingesetzt. Zusätzlich können ein Cokatalysator (z.B. Mangan- oder Kobaltsalze), eine Base, ein quartäres Salz, verschiedene Chinone bzw. Hydrochinone und Trockenmittel eingesetzt werden. Dabei kann in einem Lösungsmittel, bevorzugt Methylenchlorid, gearbeitet werden.

Bei der Umsetzung aromatischer Hydroxyverbindungen mit Kohlenmonoxid und Sauerstoff wird pro Mol gebildeten organischen Carbonats ein Mol Wasser frei. Verbleibt dieses Wasser im Reaktionssystem, kann bereits gebildetes organisches Carbonat hydrolysiert werden, so daß die erzielbaren Raum-Zeit-Ausbeuten ohne eine effektive Wasserabtrennung nur gering sind. Außerdem kann das Katalysatorsystem durch Wasser desaktiviert werden. Die Reaktivierung des desaktivierten Katalysators erfordert einen hohen technischen Aufwand. Der Ersatz des desaktivierten Katalysators ist mit hohen Kosten verbunden. Aus diesen Gründen ist eine effektive Wasserentfernung für wirtschaftliche Nutzung dieses Prozesses essentiell.

In DE-OS 27 38 437 wird zur Wasserabtrennung der Zusatz von Molsieb vorgeschlagen. Die Verwendung von Molsieb macht eine technische Nutzung des Verfahrens jedoch unattraktiv, da für eine effektive Abtrennung des Wassers aus der Flüssigphase große Mengen Molsieb (100 - 500 % Überschuß) benötigt werden, die unter hohem technischen Aufwand regeneriert werden müssen.

In EP 450 442 wird der Einsatz von Kohlendioxid als Trockenmittel vorgeschlagen. Zur Trocknung des Reaktionssystems werden dem Reaktionsgas, das aus Sauerstoff und Kohlenmonoxid besteht, ca. 30-35 % Kohlendioxid zugesetzt. Dieses Verfahren hat zwei wesentliche Nachteile: Durch die Verdünnung des Reaktionsgases wird die Raum-Zeit-Ausbeute dramatisch gesenkt. Darüber hinaus muß bei einer Kreisführung des Reaktionsgases, wie es die technische Umsetzung dieses Verfahrens erfordert, eine Anreicherung des Kohlendioxids im Gasstrom durch aufwendige Verfahren verhindert werden. Dies erfordert einen hohen apparativen Aufwand und damit verbunden hohe Kosten, die eine wirtschaftliche Nutzung dieses Verfahrens unmöglich machen.

In JP-04 257 546 wird ein Verfahren beschrieben, bei dem organische Carbonate durch oxidative Umsetzung einer aromatischen Hydroxyverbindung mit Kohlenmonoxid in Gegenwart eines Edelmetall-Katalysators und eines quartären Salzes mit einem Iodidanion durch kontinuierliches Einspeisen in eine Destillationskolonne bei 150-205°C und 30-50 bar hergestellt werden. Das Reaktionswasser wird kontinuierlich abdestilliert. Nachteilig bei diesem Verfahren ist, daß man zur Entfernung des Reaktionswassers in einer Destillationskolonne arbeiten muß, die aufgrund ihrer Konstruktion nur kurze Verweilzeiten ermöglicht. Die mit diesem Verfahren realisierbaren Raum-Zeit-Ausbeuten sind demzufolge mit nur 17,8 g/l·h sehr niedrig. Mit der Reaktionsführung in einer Destillationskolonne ist die Verwendung großer Mengen Halogenide bei hohen Temperaturen (150 - 205°C) verbunden. Dies führt zu großen Korrosionsproblemen, die zusätzlich einen hohen apparativen Aufwand bedingen. Dem Fachmann ist außerdem bekannt, daß unter den angegebenen Reaktionsbedingungen das bevorzugt eingesetzte Jodid nicht stabil ist und in erheblichem Maße zu Jod oxidiert wird. Dies führt zu großen Verlusten des quartären Salzes und zur Bildung von Nebenprodukten, was die Selektivität und damit die Wirtschaftlichkeit dieses Verfahrens stark beeinträchtigt. Bei diesen hohen Temperaturen und Drücken ist außerdem mit einer raschen Desaktivierung des homogenen Katalysatorsystems, verursacht durch die Halogenverluste und das Teilchenwachstum des Palladiums, zu rechnen, so daß eine wirtschaftliche Nutzung dieses Verfahrens nicht möglich ist.

In JP-04 261 142 wird ein Verfahren beschrieben, bei dem wie in JP-04 257 546 gearbeitet wird, jedoch mit dem Unterschied, daß an die Destillationskolonne zur Verweilzeiterhöhung zusätzliche Reaktoren angebracht wurden. Die oben angeführten Probleme der Korrosion, Katalysatordesaktivierung und des Verlustes an quartärem Salz sowie die damit verbundenen Nebenreaktionen sind auch in dieser Anmeldung nicht gelöst. Die vorgeschlagene Apparatur bringt auch sonst keine Vorteile. Die Verweilzeit wird durch die zusätzlichen Reaktoren zwar vergrößert, jedoch führt die vorgeschlagene Konstruktion zu einer erheblichen Rückvermischung innerhalb der Apparatur, so daß Nebenreaktionen in verstärktem Maße ablaufen können, wodurch die Selektivität von 99 % (JP-04 257 546) auf 97 % (JP-04 261 142) zurückgeht. Durch die zusätzlich angebrachten Reaktoren wird eine effektive Entfernung des Reaktionswassers unmöglich, denn das in den Reaktoren gebildete Reaktionswasser wird erst nachträglich in der Destillationskolonne entfernt, so daß in den Reaktoren gebildetes Carbonat hydrolytisch wieder gespalten wird. Die mit JP-04 261 142 erzielbaren Raum-Zeit-Ausbeuten sind deswegen mit ca. 9 g/l·h noch einmal deutlich geringer als bei JP-04 257 546, so daß eine wirtschaftliche Nutzung auch dieses Verfahrens nicht möglich ist.

Es bestand daher die Aufgabe, ein Verfahren zu finden, das es erlaubt, die Synthese aromatischer Carbonate unter kontinuierlicher Entfernung des entstehenden Reaktionswassers mit hoher Raum-Zeit-Ausbeute unter wirtschaftlichen, technisch realisierbaren und reproduzierbaren Bedingungen durchzuführen.

Es wurde nun gefunden, daß die beschriebenen Nachteile überwunden werden können, wenn man aus dem Reaktor einen Teil der Reaktionslösung oder die gesamte Reaktionslösung entnimmt, entspannt, aus der Reaktionslösung Wasser unter reduziertem Druck abzieht und anschließend dem Reaktor die so entwässerte Reaktionslösung wieder zuführt. Überraschenderweise erleidet das Katalysatorsystem bei dieser Entspannung bzw. Vakuumanwendung, keine Schädigung.

Die Erfindung betrifft demnach ein Verfahren zur Herstellung eines aromatischen Carbonats der Formel

R-O-CO-O-R (I),

in der
- R: substituiertes oder nicht substituiertes C₆-C₁₂-Aryl, bevorzugt substituiertes oder nicht substituiertes Phenyl, besonders bevorzugt nicht substituiertes Phenyl bedeutet,
durch Umsetzung einer aromatischen Hydroxyverbindung der Formel

R-O-H (II),

worin R die oben angegebene Bedeutung hat,
mit Kohlenmonoxid und Sauerstoff in Gegenwart eines Platinmetall-Katalysators, eines Cokatalysators, eines quartären Salzes und einer Base bei einer Temperatur von 30 bis 200°C, bevorzugt 30 bis 150°C, besonders bevorzugt 40 bis 120°C und einem Druck von 1 bis 200 bar, bevorzugt 2 bis 100 bar, besonders bevorzugt 5 bis 50 bar, bei dem ein Teil der Reaktionslösung oder die gesamte Reaktionslösung entspannt wird, bei vermindertem Druck aus der Reaktionslösung Wasser entfernt wird und die entwässerte Reaktionslösung anschließend wieder der Reaktion zurückgeführt wird.

Bevorzugt wird das in den Edukten enthaltene Wasser und das kontinuierlich durch die Reaktion entstehende Wasser durch Entnahme eines Teilstroms der Reaktionslösung entfernt, der zunächst entspannt, dann bei reduziertem Druck durch Verdampfen des Wassers entwässert und anschließend dem Reaktor wieder zugeführt wird. Besonders bevorzugt erfolgt die Wasserentfernung unter weitgehend isothermen Bedingungen.

Der pro Stunde entnommene Teilstrom an Reaktionslösung kann im erfindungsgemäßen Verfahren das 0,01- bis 30fache, bevorzugt das 0,05- bis 20fache, besonders bevorzugt das 0,1- bis 10fache des Reaktorinhalts betragen.

Die Verdampfung des Wassers kann in Apparaten erfolgen, die dem Fachmann bekannt sind, wie z.B. in Vertikalrohr-, Horizontalrohr-, Schrägrohr-, Rotor- bzw. Dünnschicht-, Zentrifugal-, Schnecken- und Fallfilmverdampfern, in Rohrbündel-, Rohrkorbverdampfern, Verdampfern mit außenliegendem Rücklaufrohr und Zwangsumlauf, Verdampfern mit außenliegendem Heizkörper und Zwangsumlauf und weiteren dem Fachmann bekannte Verdampfern (P. Grassmann, F. Widmer, Einführung in die thermische Verfahrenstechnik, Walter de Gruyter, Berlin, New York 1974); weiterhin sind auch begleitbeheizte einfache Destillations- und Rektifizierkolonnen geeignet; bevorzugt einzusetzen sind Dünnschicht- und Fallfilmverdampfer und Verdampfer mit Zwangsumlauf und innen oder außen liegenden Heizregistern.

In einer weiteren Ausführungsform läßt sich die Wasserentfernung durch spontane Verdampfung bei der Entspannung auf reduzierten Druck realisieren, hierbei kühlt sich der entnommene Teilstrom ab und muß vor der Rückführung wieder auf Reaktionstemperatur erwärmt werden.

Das erfindungsgemäße Verfahren zur Carbonatbildung wird bei einer Reaktionstemperatur von 30 bis 200°C, bevorzugt 30 bis 150°C, besonders bevorzugt 40 bis 120°C, und bei einem Reaktionsdruck von 1 bis 200 bar, bevorzugt 2 bis 100 bar, besonders bevorzugt 5 bis 50 bar durchgeführt. Zur Wasserentfernung im erfindungsgemäßen Verfahren wird eine Temperatur eingestellt, die im Bereich von bis zu ± 30°C, bevorzugt bis zu ± 20°C, besonders bevorzugt bis zu ± 10°C der Reaktionstemperatur liegt. Der Druck für die Wasserentfernung liegt bei 1 bis 5 000 mbar, bevorzugt 2 bis 3 000 mbar, besonders bevorzugt 5 bis 1 000 mbar.

In der bevorzugten Ausführungsform sind alle Rohrleitungen und Verdampfer derart beheizt, daß die entnommene Reaktionslösung weitgehend auf der Temperatur des Reaktorinhalts bleibt.

Durch ein im Abgasstrom der Entwässerung befindliches Trennorgan, wie z.B. einen Dephlegmator, eine Destillationskolonne mit Böden oder Packung und weitere dem Fachmann bekannte Apparate, kann der größte Teil der bei der Entwässerung mitgerissenen Edukte (z.B. Phenol) und Produkte vom Wasser abgetrennt und in den Rückstrom zum Reaktor geführt werden. Die Trennung des abgetrennten Gemisches aus Wasser und mitgerissenen Edukten oder Produkten kann nach dem Stand der Technik z.B. durch Extraktion oder Destillation erfolgen.

Der mit dem Gemisch aus Wasser und mitgerissenen Edukten oder Produkten ausgetriebene Anteil an gelösten Gasen läßt sich in einer bevorzugten Ausführungsform nach der Abtrennung wieder dem Reaktorkreisgas zuführen. Die Abtrennung mitgerissener Edukte (z.B. Phenol), Produkte und Wasser aus dem ggf. vor der Trennung komprimierten zu recyclisierenden Gasgemisch erfolgt nach dem Stand der Technik, z.B. durch Adsorption, Absorption oder bevorzugt durch Kondensation. Das zur Reaktion benötigte Reaktionsgas, bestehend aus Kohlenmonoxid, Sauerstoff und einem Inertgas, wird hierzu in einer Menge von 1 bis 10000 Nl pro Liter Reaktionslösung, bevorzugt 5 bis 5000 Nl pro Liter Reaktionslösung und besonders bevorzugt 10 bis 1000 Nl pro Liter Reaktionslösung eingeleitet. Das aus der Entwässerung stammende, zu recyclisierende Gas wird bezüglich seiner Anteile an CO und O₂ auf die genannten Volumina angerechnet.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,001-1,0) bevorzugt 1:(0,01-0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen. So kann Synthesegas als CO-Quelle und Luft als O₂-Träger dienen, es ist jedoch darauf zu achten, daß keine Katalysatorgifte wie z.B.: Schwefel oder dessen Verbindungen eingetragen werden. In der bevorzugten Ausführungsform des erfindungsgemäßen Verfahrens werden reines CO und reiner Sauerstoff verwendet.

Inerte Bestandteile der Reaktionsgase im erfindungsgemäßen Verfahren können Stickstoff, Wasserstoff, Kohlendioxid, Edelgase sowie unter Reaktionsbedingungen beständige organische Verbindungen, die mit Wasser ein Azeotrop bilden, sein. Die Konzentration an Inertgas im Reaktionsgas kann 0 bis 60 Vol.%, bevorzugt 0 bis 20, besonders bevorzugt 0 bis 5 Vol.% betragen. Die Konzentration 0 Volumen % stellt den Spezialfall des bevorzugten inertgasfreien Zustands dar.

Bei den erfindungsgemäß umsetzbaren aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von C₁-C₄-Alkyl, C₁-C₄-Alkoxy, Fluor, Chlor oder Brom.

In das erfindungsgemäße Verfahren einsetzbare Basen stellen Alkalisalze von aromatischen Hydroxyverbindungen der Formel (II) dar, in der R die oben angegebene Bedeutung hat. Ganz besonders bevorzugt wird ein Alkalisalz der aromatischen Hydroxyverbindung verwendet, die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium-, und Kaliumphenolate, besonders bevorzugt Natriumphenolat eingesetzt.

Das Alkalimetallphenolat kann dem Reaktionsgemisch als reine Verbindung in fester Form oder als Schmelze zugesetzt werden. Selbstverständlich können in das erfindungsgemäße Verfahren auch die Hydrate der Alkalimetallphenolate eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen im allgemeinen zu schlechteren Umsätzen und Zersetzung gebildeter Carbonate. In einer weiteren Ausführungsform der Erfindung wird das Alkalimetallphenolat dem Reaktionsgemisch als Lösung, die 0,1 - 80 Gew.-%, bevorzugt 0,5 - 65 Gew.-%, besonders bevorzugt 1 - 50 Gew.-% des Alkalimetallphenolats enthält, zugesetzt. Als Lösungsmittel können hierbei sowohl Alkohole oder Phenole, wie z.B. das umzusetzende Phenol, als auch inerte Lösungsmittel verwendet werden. Als solche seien die weiter unten als Reaktionsmedien erwähnten genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem inerten Lösungsmittel verdünnt wurde. Bevorzugt wird die Base in der Schmelze einer aromatischen Hydroxyverbindung gelöst. Besonders bevorzugt wird die Base in einer Schmelze der aromatischen Hydroxyverbindung gelöst, die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall, z.B. Palladium, zu Base wird vorzugsweise so gewählt, daß pro Grammatom Platinmetall, z.B. Palladium, 0,1 bis 500, bevorzugt 0,3 bis 200 besonders bevorzugt 0,9 bis 130 Äquivalente Base eingesetzt werden.

Das erfindungsgemäße Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether, wie Dioxan, Tetrahydrofuran, t-Butyl-methylether und veretherte Glykole, genannt.

Die für das erfindungsgemäße Verfahren geeigneten Platinmetall-Katalysatoren bestehen aus mindestens einem Edelmetall der Gruppe VIII, vorzugsweise Palladium. Es kann bei dem erfindungsgemäßen Verfahren in verschiedener Form zugegeben werden. Palladium kann in metallischer Form oder bevorzugt in Form von Palladium-Verbindungen der Oxidationsstufen 0 und +2, wie beispielsweise Palladium(II)-acetylacetonat, -halogenide, -carboxylate von C₂-C₆-Carbonsäuren, -nitrat, -oxide oder Palladiumkomplexe, die beispielsweise Olefine, Amine, Phosphine und Halogenide enthalten können, eingesetzt werden. Besonders bevorzugt sind Palladiumbromid und Palladiumacetylacetonat.

Die Menge an Platinmetall-Katalysator ist im erfindungsgemäßen Verfahren nicht beschränkt. Bevorzugt wird so viel Katalysator zugesetzt, daß die Konzentration des Metalls im Reaktionsansatz 1 - 3000 ppm beträgt, besonders bevorzugt sind Konzentrationen von 5 - 500 ppm.

Als Cokatalysator für das erfindungsgemäße Verfahren wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew) eingesetzt, wobei das Metall in verschiedenen Oxidationsstufen eingesetzt werden kann. Bevorzugt werden Mn, Cu, Co, V, Zn, Ce und Mo eingesetzt. Ohne das erfindungsgemäße Verfahren einzuschränken, seien Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₆-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Besonders bevorzugt werden Mn, Cu, Mo und Ce eingesetzt. Ganz besonders bevorzugt werden Manganverbindungen im erfindungsgemäßen Verfahren verwendet, besonders bevorzugt Mangan(II)komplexe, ganz besonders bevorzugt Mangan(II)acetylacetonat bzw. Mangan(III)-acetylacetonat.

Der Cokatalysator wird in einer solchen Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quartären Salzen kann es sich beispielsweise um mit organischen Resten substituierte Ammonium-, Phosphonium- oder Sulfoniumsalze handeln. Geeignet für den Einsatz in das erfindungsgemäße Verfahren sind Ammonium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat tragen. Bevorzugt werden in das erfindungsgemäße Verfahren Ammoniumsalze eingesetzt, die als organische Reste C₆- bis C₁₀-Aryl-, C₇- bis C₁₂-Aralkyl- und/oder C₁- bis C₂₀-Alkyl-Reste und als Anion ein Halogenid tragen, besonders bevorzugt ist Tetrabutylammoniumbromid. Die Menge eines solchen quartären Salzes kann beispielsweise 0,1 - 20 Gew.-%, bezogen auf das Gewicht des Reaktionsgemisches, betragen. Vorzugsweise beträgt diese Menge 0,5 - 15 Gew.-%, besonders bevorzugt 1 - 5 Gew.-%.

Vor dem Einsatz in das erfindungsgemäße Verfahren wird in bevorzugter Weise der Platinmetall-Katalysator aktiviert. Dazu wird die Platinmetall-Verbindung bevorzugt so bemessen, daß ihre Konzentration im Aktivierungsansatz 0,0001 bis 30 Gew.-%, besonders bevorzugt 0,001 bis 10 Gew.-% beträgt, und in einem inerten Lösungsmittel oder direkt in der Schmelze der aromatischen Hydroxyverbindung oder Mischungen derselben gelöst. Zu dieser Lösung wird das obige quartäre Salz gegeben. Diese Lösung wird anschließend bei 15 bis 200°C, bevorzugt bei 20 bis 150°C, besonders bevorzugt bei 40 bis 100°C mit Kohlenmonoxid behandelt. Das kann sowohl dadurch geschehen, daß man bei Normaldruck pro Gramm des eingesetzten Platinmetalls Kohlenmonoxid in einer Menge von 0,1 bis 250 l/h, bevorzugt 0,5 bis 200 l/h, besonders bevorzugt 1 bis 100 l/h einleitet, als auch dadurch, daß man die Lösung in einem Autoklaven unter einem Druck von 1 bis 300 bar, bevorzugt 1 bis 200 bar, besonders bevorzugt 1-150 bar mit Kohlenmonoxid versetzt. Die Aktivierungszeit hängt vom verwendeten Platinmetall-Katalysator und von einem ggf. eingesetzten inerten Lösungsmittel ab. Sie beträgt im allgemeinen wenige Minuten bis einige Stunden. Der Platinmetall-Katalysator kann direkt vor der Reaktion aktiviert werden, aber auch nach Abtrennung des Lösungsmittels oder der aromatischen Hydroxyverbindung, z.B. durch Abdestillieren, in aktivierter Form isoliert und gelagert werden.

In einer weiteren bevorzugten Ausführungsform werden statt des homogenen Katalysatorsystems heterogene Katalysatoren, bei denen das Platinmetall oder das Platinmetall und der Cokatalysator auf einem heterogenen Träger aufgebracht sind, als Pulver oder Formkörper eingesetzt. Die übrigen Komponenten des Katalysatorsystems, wie die Base, die quartäre Verbindung und gegebenenfalls der Cokatalysator, sind weiterhin in der Reaktionslösung homogen gelöst. Die Menge des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der oben genannten Art eingesetzt.

Die Menge des Cokatalysators am Gesamtgewicht des heterogenen Katalysators beträgt 0,01 bis 15 Gew.-%, bevorzugt 0,05 bis 10 Gew.-% gerechnet als Metall.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch sowie Eisen- und Kobaltoxide, Nickel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Trägerkatalysator-Pulver auf die eingesetzte Menge an aromatischer Hydroxyverbindung, verwendet.

In besonders bevorzugten Ausführungsformen wird der heterogene Trägerkatalysator ortsfest in Rührbehältern, Blasensäulen, einem Rieselphasenreaktor oder Kaskaden dieser Reaktoren eingesetzt. Eine Abtrennung des Trägerkatalysators entfällt dann völlig.

Die Herstellung von aromatischen Carbonaten mit den erfindungsgemäßen Platinmetall-Trägerkatalysatoren kann diskontinuierlich oder kontinuierlich erfolgen. Im Falle einer kontinuierlichen Fahrweise im Gegen- oder Gleichstrom oder in der Rieselphase am Festbett-Katalysator werden Belastungen von 0,01 bis 20 g aromatische Hydroxyverbindung pro Gramm Trägerkatalysator und Stunde, bevorzugt 0,05 bis 10 g aromatische Hydroxyverbindung pro Gramm Trägerkatalysator und Stunde, besonders bevorzugt 0,1 bis 5 g aromatische Hydroxyverbindung pro Gramm Trägerkatalysator und Stunde eingestellt.

Als Reaktoren für das erfindungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß zu verwenden Rührbehälter mit dafür brauchbaren Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können in dem erfindungsgemäßen Verfahren folgende Typen, eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 51 (1979) Nr. 3, S. 208-216; W.-D. Deckwer, Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer, im Falle eines Rührkessels oder andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet.

Im Falle der Verwendung pulverförmiger Trägerkatalysatoren können diese aus der Reaktionsmischung z.B. durch Filtration, Sedimentation oder Zentrifugieren abgetrennt werden.

In diskontinuierlichen Versuchen verwendete Trägerkatalysatoren können bei gleichen Einsatzstoffen ggf. ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch ggf. regeneriert werden.

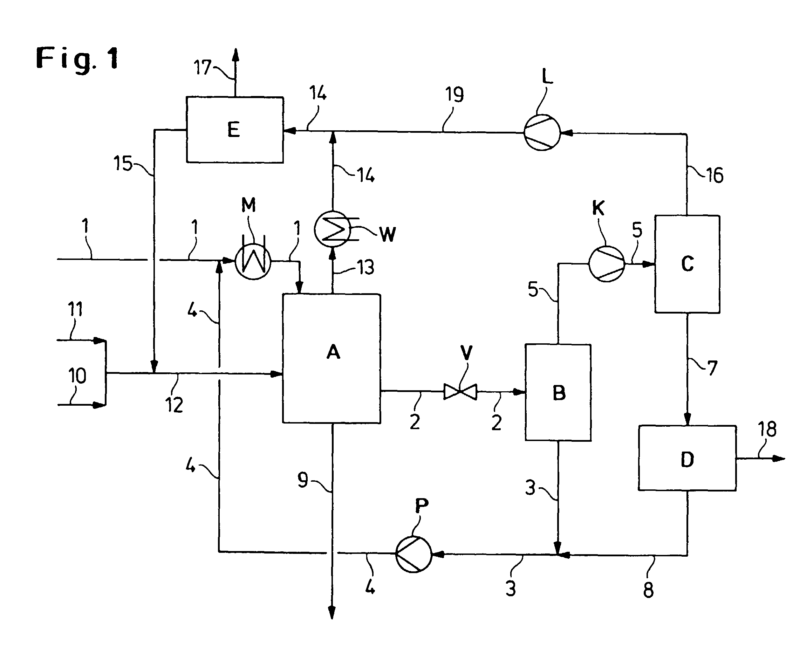

In Figur 1 ist beispielhaft die Arbeitsweise mit nur einem Reaktor dargestellt, wobei die erfindungsgemäße Arbeitsweise nicht auf dieses Beispiele eingeschränkt werden soll. Die bevorzugte Ausführungsform des erfindungsgemäßen Verfahrens besteht darin, daß man den Platinmetall-Katalysator in der oben beschriebenen Weise aktiviert und diese Lösung dann gleichzeitig mit weiteren Lösungen, die die restlichen Komponenten des Reaktionssystems in den oben angegebenen Konzentrationen enthalten, kontinuierlich über Leitung 1 dem Reaktor (**A**) zusetzt. Durch ein vorgeschaltetes Erhitzerelement (**M**) lassen sich die flüssigen Reaktionskomponenten auf die vorgesehene Reaktionstemperatur vorheizen. Es kann gegebenenfalls von Vorteil sein, die zur Reaktion notwendige Base separat zu dosieren. Ein Teilstrom des Reaktorinhalts wird kontinuierlich über Leitung 2 und das Entspannungsventil (**V**) dem Verdampfer (**B**) zugeführt. Die entwässerte Lösung wird über Leitung 3, die Pumpe (**P**) und Leitung 4 entweder direkt oder über Leitung 1 und das Erhitzerelement (**M**) dem Reaktor wieder zugeführt. Das im Vakuum verdampfte Wasser-Phenol-Gemisch verläßt über Leitung 5 den Verdampfer (**B**), wird mit Hilfe des Verdichters (**K**) wieder verdichtet; im Abscheider (**C**) wird das Wasser-Phenol-Gemisch vom mitgeschleppten Reaktionsgas abgetrennt, das über Leitung 16 dem Verdichter (**L**) und Leitung 19 der CO₂-Abtrennung (**E**) zugeführt wird. Das Wasser-Phenol-Gemisch gelangt über Leitung 7 in die Wasser-Phenol-Trennung (**D**). Über Leitung 8 wird zurückgewonnenes Phenol dem Verdampferablauf 3 zugeführt und das abgetrennte Wasser über Leitung 18 ausgeschleust. Kohlenmonoxid und Sauerstoff gelangen über Leitungen 10 bzw. 11 und Leitung 12 in den Reaktor (**A**). Dabei werden CO und Sauerstoff, in den oben angegebenen Mengen, entweder durch einen Begasungsrührer, im Falle eines Rührkessels oder andere bekannte Gasverteilungsorgane in die Reaktionsmischung geleitet. Überschüssiges Reaktionsgas verläßt über Leitung 13 den Reaktor, wird mit Hilfe des Kondensators (**W**) von mitgerissenem Phenol befreit und gelangt über Leitung 14 in die CO₂-Abtrennung (**E**). Über Leitung 15 gelangt überschüssiges Reaktionsgas zusammen mit dem Feedgas wieder in den Reaktor (**A**). Über Leitung 17 wird CO₂ ausgeschleust. Das Reaktionsprodukt wird über Leitung 9 entnommen und der Aufarbeitung zugeführt. Die Füllstandsregelung im kontinuierlich betriebenen Reaktor erfolgt nach bekannten Methoden.

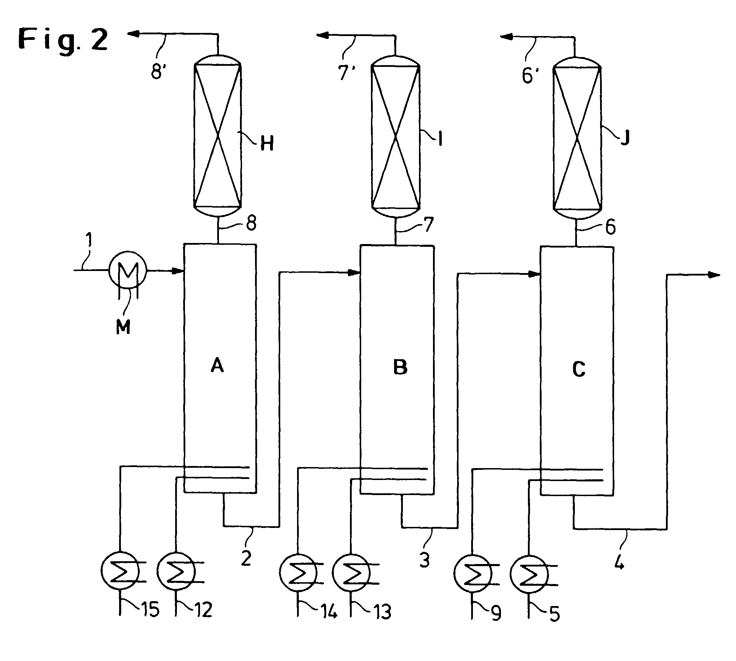

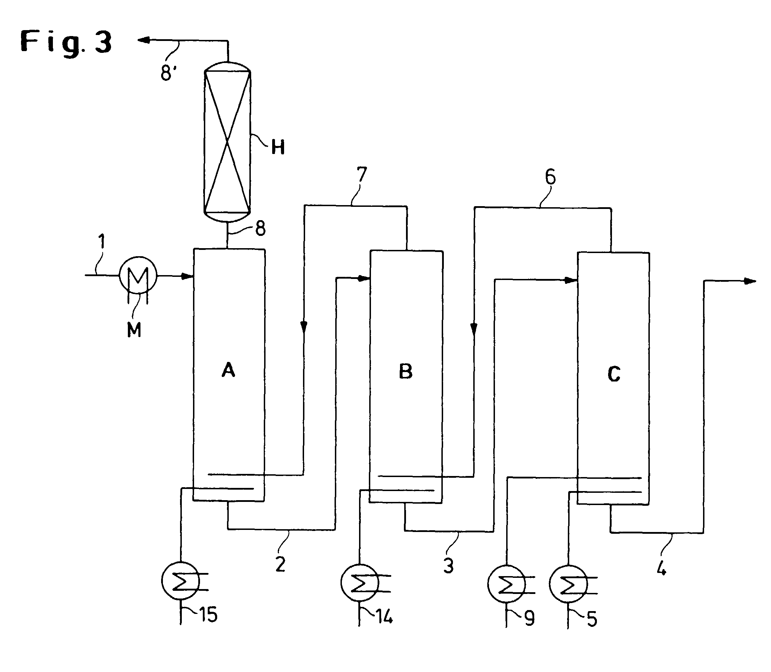

Im Falle der Verwendung einer Reaktorkaskade (Figuren 2 und 3, hier beispielsweise mit jeweils 3 Reaktoren dargestellt) werden die oben beschriebenen flüssigen Reaktionskomponenten in den ersten Reaktor (**A**) eindosiert und können gegebenenfalls in einem vorgeschalteten Erhitzerelement (**M**) auf die vorgesehene Reaktionstemperatur vorgeheizt werden. Sie werden in flüssiger Form über Leitung 1 bevorzugt am oberen Ende des Reaktors eingebracht. Die aus den jeweiligen Reaktoren zu entnehmende Flüssigphase wird am unteren Reaktorende entnommen und über die Leitungen 2 bzw. 3 in den jeweils folgenden Reaktor am oberen Ende wieder eindosiert. Am letzten Reaktor wird der Produktstrom entnommen (Leitung 4 in Figuren 2 u. 3) und der Aufarbeitung zugeführt. Die Regelung des gewünschten Füllstandes in den kontinuierlich betriebenen Reaktoren erfolgt nach bekannten Methoden. Die erfindungsgemäße Entwässerung kann an jedem Kaskadenreaktor erfolgen, wobei die Phenolrückführung in jeden Einzelreaktor oder bevorzugt in den ersten Reaktor erfolgen kann. Die Rückführung der entwässerten Reaktionslösungen erfolgt bevorzugt in den jeweiligen Reaktor, weniger bevorzugt in den ersten Reaktor. Die Phenol-Wasser-Kondensation und anschließende Auftrennung in Phenol und Wasser kann an jedem Einzelreaktor oder bevorzugt zentral in einem Einzelaggregat erfolgen. Die Gasrückführung aus der oben beschriebenen Wasserabtrennung kann ebenfalls zentral, zusammen mit dem Kreislaufgas und der Gasergänzung, erfolgen.

Bei der Benutzung einer Kaskade kann die Gasphase durch den kontinuierlich laufenden Flüssigkeitsstrom entweder im Querstrom (Fig. 2) oder im Gegenstrom (Fig. 3) geschickt werden. Zur besseren Übersichtlichkeit ist in diesen Figuren das erfindungsgemäße Verfahren zur Wasserabtrennung nicht eingezeichnet, um die Anwendung von Quer- und Gegenstrom besser verdeutlichen zu können. Es sollen hierdurch weitere Varianten des erfindungsgemäße Verfahren beschrieben werden, ohne es jedoch auf diese einzuschränken.

Querstromfahrweise der Kaskade bedeutet hierbei, daß die Reaktionsgase über die Leitungen 12, 15, 13, 14, 9 und 5 (Fig. 2) eindosiert werden und jeweils am oberen Ende eines jeden Reaktors über die Leitungen 8, 7 und 6 (Fig. 2) zusammen mit dem mitgerissenem Edukt (II) wieder entnommen werden, d.h. das Reaktionsgas durchströmt die Reaktoren quer zur Flußrichtung der Flüssigphase. In den Trennorganen (**H**), (**I**) und (**J**) wird das Edukts (II) abgetrennt und in die jeweiligen Reaktoren zurückgeführt. Über die Leitungen 8', 7' und 6' verläßt das überschüssige Reaktionsgas den Reaktor und wird nach der CO₂-Abtrennung sowie dem Ersatz des verbrauchten Reaktionsgases den Reaktoren (**A**), (**B**) und (**C**) wieder zugeführt. Die O₂-Ergänzung kann hierbei zentral oder über die Leitungen 15, 14 und 9 erfolgen, wobei die Gesamtmenge des eindosierten Sauerstoffs beliebig auf die einzelnen Reaktoren aufgeteilt werden kann. Ebenso kann die Gesamtmenge des eindosierten Kohlenmonoxids beliebig auf die einzelnen Reaktoren aufgeteilt werden. In jedem Einzelreaktor wird hierdurch im Gegensatz zur Gesamtkaskade die Gegenstromfahrweise von Flüssigphase und Gasphase realisiert.

Gegenstromfahrweise der Kaskade (Fig. 3) bedeutet, daß man die Gasphase in den letzten Reaktor (**C**) eindosiert, kontinuierlich gegen die vom ersten Reaktor (**A**) zum letzten Reaktor (**C**) laufenden Flüssigphase durch die Leitungen 6 und 7 (Fig. 3) führt und am jeweils unteren Ende des Reaktors (**B**) bzw. (**A**) wieder einleitet. Die Dosierung und Führung der Flüssigphase in den Reaktoren ist mit der Querstromfahrweise identisch. Am oberen Ende des ersten Reaktors (**A**) wird das überschüssige Reaktionsgas zusammen mit mitgerissenem Edukt (II) über Leitung 8 entnommen. Im Trennorgan (**H**) wird das Edukt (II) abgetrennt und in den Reaktor (**A**) zurückgeführt. Über die Leitung 8' verläßt das überschüssige Reaktionsgas den Reaktor und wird nach der CO₂-Abtrennung sowie dem Ersatz des verbrauchten Reaktionsgases dem Reaktor (**C**) wieder zugeführt. Zur Gewährleistung eines konstanten O₂-Gehalts kann es zweckmäßig sein, über die Leitungen 14, 15 und 9 Sauerstoff getrennt vom Kohlenmonoxid in die Reaktoren einzuleiten, wobei die Gesamtmenge des eindosierten Sauerstoffs beliebig auf die einzelnen Reaktoren aufgeteilt werden kann.

Die in den Figuren 1-3 beispielhaft an Blasensäulen beschriebenen Ausführungen lassen sich ohne Einschränkungen auf einen Rührkessel bzw. Rührkesselkaskaden mit geeigneten Rührorganen übertragen.

Die Aufarbeitung des flüssigen Reaktionsprodukts erfolgt nach dem Stand der Technik durch Filtration, Destillation und Kristallisation. Die zurückgewonnenen Katalysatorkomponenten werden recyclisiert, indem man ausgeschleuste Anteile ergänzt und die Katalysatorkomponenten in aktiver Form, zusammen mit Kreislaufphenol und Frischphenol, dem Reaktor (**A**) wieder zugeführt.

Die folgenden Beispiele verdeutlichen das erfindungsgemäße Verfahren, ohne es jedoch auf diese einzuschränken.

### Beispiel 1:

In einem Autoklaven (1 l) mit Begasungsrührer, Kühler und nachgeschalteter Kühlfalle sowie einer zuschaltbaren Evakuierungslinie mit Tiefkühlfallen, wurden 0,34 g Palladiumbromid und 8,31 g Tetrabutylammoniumbromid bei 80°C in 450 g Phenol gelöst. Durch diese Lösung wurde zur Aktivierung des Katalysators eine Stunde lang Kohlenmonoxid (3 l/h) geleitet. Dann wurden 0,77 g Mangan(II)acetylacetonat und 2,21 g Natriumphenolat, gelöst in 50 g Phenol, zugegeben und unter Einleitung eines Gasgemisch aus Kohlenmonoxid und Sauerstoff (96,5:3,5 Vol.%) der Druck auf 10 bar eingestellt. Die Menge an Gasgemisch, bestehend aus Kohlenmonoxid und Sauerstoff, wurde auf 80 Nl/h eingestellt. Dem Reaktionsgemisch wurde jede Stunde eine Probe entnommen und gaschromatographisch analysiert. Nach der Probenahme wurde der Autoklav zur Wasserentfernung entspannt, die Dosierung der Gase durch Schließen der Ventile abgestellt und für 5 min ein Vakuum von 20 mbar angelegt, anschließend der Druck wieder mit dem Kohlenmonoxid/Sauerstoffgemisch auf 10 bar erhöht und die Reaktion wie oben beschrieben fortgesetzt. Dieser Zyklus wurde insgesamt 3 mal wiederholt.

Die Analysen ergaben, daß nach einer Stunde 7,6% Diphenylcarbonat, nach 2 Stunden 12,4% Diphenylcarbonat und nach 3 Stunden 17,7% Diphenylcarbonat im Reaktionsgemisch enthalten waren. In den Tiefkühlfallen der Vakuumlinie waren 22,7 g eines Phenol/Wasser-Gemisches kondensiert.

### Vergleichsbeispiel:

Der Versuch wurde, wie in Beispiel 1 beschrieben, wiederholt, es wurde jedoch auf die Evakuierungszyklen zur Wasserentfernung verzichtet. Die gaschromatographischen Analysen der Proben ergaben, daß nach einer Stunde 4,7% Diphenylcarbonat, nach 2 Stunden 5,9% Diphenylcarbonat und nach 3 Stunden 6,4% Diphenylcarbonat im Reaktionsgemisch enthalten waren. In der Kühlfalle hinter dem Kühler waren 0,2 g eines Phenol/Wasser-Gemisches kondensiert.

### Beispiel 2:

Der Versuch wurde, wie in Beispiel 1 beschrieben, wiederholt, es wurden jedoch statt 0,34 nur 0,08 g PdBr₂ eingesetzt.

Die Analysen ergaben, daß nach einer Stunde 7,3% Diphenylcarbonat, nach 2 Stunden 12,2% Diphenylcarbonat und nach 3 Stunden 17,1% Diphenylcarbonat im Reaktionsgemisch enthalten waren. In den Tiefkühlfallen der Vakuumlinie waren 26,0 g eines Phenol/Wasser-Gemisches kondensiert.

### Beispiel 3:

Das Beispiel 1 wurde wiederholt, wobei statt 0,34 g PdBr₂ 0,10 g Palladiumacetylacetonat eingesetzt wurden. Mangan(II)acetylacetonat wurde bereits vor der Aktivierung des Palladiums zugesetzt.

Die Analysen ergaben, daß nach einer Stunde 8,5 % Diphenylcarbonat, nach 2 Stunden 13,2% Diphenylcarbonat und nach 3 Stunden 18,5 % Diphenylcarbonat im Reaktionsgemisch enthalten waren. In den Tiefkühlfallen der Vakuumlinie waren 24,9 g eines Phenol/Wasser-Gemisches kondensiert.

### Beispiel 4:

Das Beispiel 1 wurde wiederholt, wobei statt 0,34 g nur 0,08 g Palladiumbromid eingesetzt wurden. Mangan(II)acetylacetonat wurde bereits vor der Aktivierung des Palladiums zugesetzt.

Die Analysen ergaben, daß nach einer Stunde 8,1 % Diphenylcarbonat, nach 2 Stunden 13,4% Diphenylcarbonat und nach 3 Stunden 18,7 % Diphenylcarbonat im Reaktionsgemisch enthalten waren. In den Tiefkühlfallen der Vakuumlinie waren 25,4 g eines Phenol/Wasser-Gemisches kondensiert.

### Beispiel 5:

### Belegung eines pulverförmigen Titandioxids mit Palladium und Mangan:

Zu einer Aufschlämmung von 283,5 g Titandioxid-Pulver (Norton) in 1500 ml Wasser wurden bei Raumtemperatur 300 ml Lösung von 40,5 g (0,16 mol) Mangan(II)-nitrat-4-hydrat in Wasser gegeben. Dann wurde mit verdünnter Natronlauge alkalisch gestellt. Die Suspension wurde abgesaugt, mit Wasser gewaschen, bei 100°C getrocknet und 3h bei 300°C getempert. Der mit Mangan dotierte Träger wurde in 1500 ml Wasser aufgeschlämmt und mit 300 ml Lösung, enthaltend 50 g Natriumtetrachloropalladat(II)-Lösung mit 15% Palladium, versetzt. Dann wurde mit verdünnter Natronlauge alkalisch gestellt. Die Suspension wurde abgesaugt, gewaschen und bei 100°C getrocknet. Der Katalysator enthielt 2,5 % Pd und 3 % Mn, jeweils als Metall gerechnet.

### Einsatz des Trägerkatalysators zur Herstellung von Diphenylcarbonat:

Das Beispiel 1 wurde wiederholt, wobei statt 0,34 g Palladiumbromid 4 g des oben hergestellten heterogenen Katalysators eingesetzt und bei 8 bar gearbeitet wurde.

Die Analysen ergaben, daß nach einer Stunde 7,5 % Diphenylcarbonat, nach 2 Stunden 12,5 % Diphenylcarbonat und nach 3 Stunden 16,5 % Diphenylcarbonat im Reaktionsgemisch enthalten waren. In den Tiefkühlfallen der Vakuumlinie waren 23,7 g eines Phenol/Wasser-Gemisches kondensiert.

### Beispiel 6:

### Belegung eines pulverförmigen Titandioxids mit Palladium und Kobalt:

Zu einer Lösung von 18,75 g Palladium(II)-bromid (0,07 mol), 28,5 g Natriumbromid (0,28 mol) und 33,4 g Kobalt(II)-bromid (0,15 mol) in 1500 ml Wasser wurden bei Raumtemperatur 283,5 g Titandioxid-Pulver (Norton) gegeben. Dann wurde mit verdünnter Natronlauge alkalisch gestellt. Die Suspension wurde abgesaugt, gewaschen und bei 100°C getrocknet. Der Katalysator enthielt 2,5 % Pd und 3 % Co, jeweils als Metall gerechnet.

### Einsatz des Trägerkatalysators zur Herstellung von Diphenylcarbonat:

Das Beispiel 1 wurde wiederholt, wobei statt 0,34 g Palladiumbromid 4 g des oben hergestellten heterogenen Katalysators eingesetzt und bei 8 bar gearbeitet wurde.

Die Analysen ergaben, daß nach einer Stunde 6,5 % Diphenylcarbonat, nach 2 Stunden 11,3 % Diphenylcarbonat und nach 3 Stunden 15,6 % Diphenylcarbonat im Reaktionsgemisch enthalten waren. In den Tiefkühlfallen der Vakuumlinie waren 22,9 g eines Phenol/Wasser-Gemisches kondensiert.

### Beispiel 7:

### Belegung eines Titandioxid-Extrudats mit Palladium und Mangan:

200 ml Titandioxid-Extrudat wurden mit 58,4 ml Lösung von 21,6 g Mangan(II)chlorid in Wasser getränkt. Dann wurde unter Stickstoff bei 110°C getrocknet. Der mit Mangan dotierte Träger wurde mit 58 ml Lösung, enthaltend 33,3 g Natriumtetrachloropalladat(II)-Lösung mit 15% Palladium, getränkt. Dann wurde unter Stickstoff bei 110°C getrocknet. Der fertige Kontakt enthielt pro Liter 25 g Pd und 30 g Mn, jeweils als Metall gerechnet.

### Einsatz des Trägerkatalysators zur Herstellung von Diphenylcarbonat:

Der Einsatz des Trägerkatalysators zur Herstellung von Diphenylcarbonat erfolgte wie in Beispiel 6, mit dem Unterschied, daß sich der Katalysator (12ml) ortsfest in einem Maschendrahtkörbchen befand.

Die Analysen ergaben, daß nach einer Stunde 5,6 % Diphenylcarbonat, nach 2 Stunden 10,9 % Diphenylcarbonat und nach 3 Stunden 14,1 % Diphenylcarbonat im Reaktionsgemisch enthalten waren. In der Kühlfalle waren 20,7 g eines Phenol/Wasser-Gemisches kondensiert.

### Beispiel 8:

### Belegung eines Titandioxid-Extrudats mit Palladium, Kupfer und Molybdän:

200 ml Titandioxid-Extrudate wurden mit 100 ml 25%iger Ammoniak-Lösung vorgetränkt. Anschließend wurde der Träger mit einer Lösung, bestehend aus 300 ml 25%iger Ammoniak-Lösung, 1,44 g Palladium(II)-chlorid (0,008 mol), 2,76 g Kupfer(II)-chlorid-2-hydrat (0,016 mol) und 3,04 g Ammoniummolybdat(VI)-4-hydrat (0,0025 mol), versetzt.

Die Mischung wurde 1h bei 80°C gerollt und die flüchtigen Bestandteile anschließend bei 80°C im Vakuum abgezogen. Nach Trocknung unter Stickstoff bei 200°C erhielt man einen Katalysator, der 4,3 g Pd, 5,2 g Cu und 8,3 g Mo pro Liter Kontaktmasse enthielt.

### Einsatz des Trägerkatalysators zur Herstellung von Diphenylcarbonat:

Der Einsatz des Trägerkatalysators zur Herstellung von Diphenylcarbonat erfolgte wie in Beispiel 6.

Die Analysen ergaben, daß nach einer Stunde 4,5 % Diphenylcarbonat, nach 2 Stunden 6,8 % Diphenylcarbonat und nach 3 Stunden 8,7 % Diphenylcarbonat im Reaktionsgemisch enthalten waren. In der Kühlfalle waren 15,3 g eines Phenol/Wasser-Gemisches kondensiert.

### Beispiel 9:

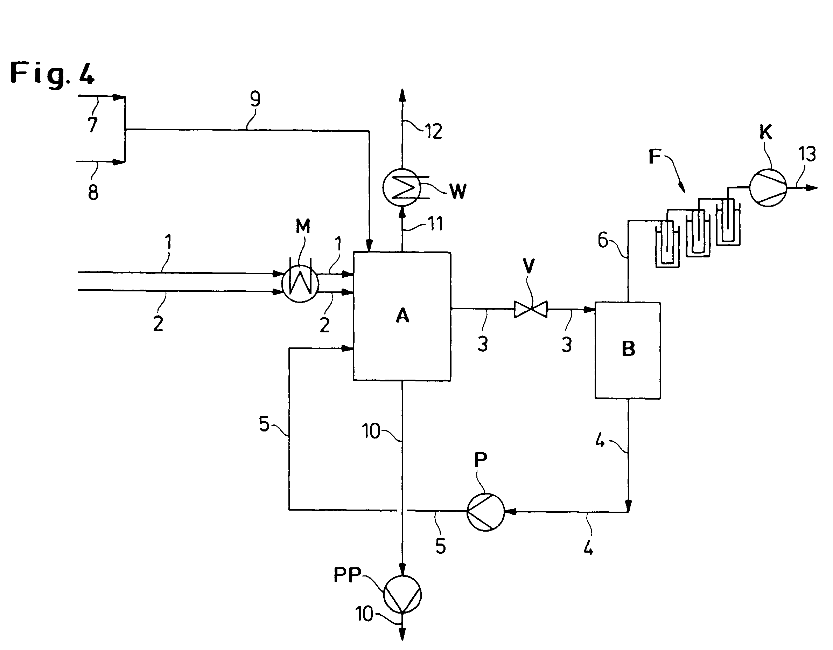

Mit der in Figur 4 schematisch dargestellten Apparatur, bestehend aus einem 1 l Autoklaven (A), kontinuierlicher Gas- und Eduktdosierung, kontinuierlicher Gas- und Flüssigkeitsausschleusung sowie kontinuierlicher Teilstromentnahme-, -behandlung und -rückführung wurde ein kontinuierlicher Versuch durchgeführt.

Über Leitung 1 und Erhitzer (M) wurde eine aktivierte Katalysatorlösung, bestehend aus 0,10 g Palladiumbromid, 8,31 g Tetrabutylammoniumbromid und 1,07 g Mangan-(III)-acetylacetonat in 450 g Phenol, stündlich in den Reaktor gefördert. Gleichzeitig wurde eine Natriumphenolatlösung, bestehend aus 2,21 g Natriumphenolat, gelöst in 50 g Phenol, stündlich über Leitung 2 und Erhitzer (M) zudosiert.

Weiterhin wurden stündlich ca. 200 ml entwässerte Reaktionslösung über Pumpe (P) und Leitung 5 zurückgeführt.

Die Temperatur der Reaktionslösungen betrug 80°C. In den Reaktor wurden über die Leitungen 7, 8 und 9 stündlich 100 Nl Gasgemisch, bestehend aus Kohlenmonoxid und Sauerstoff (96,5:3,5 Vol.%), eingeleitet. Der Reaktordruck betrug 10 bar und die Innentemperatur wurde auf 80°C geregelt. Überschüssiges Reaktionsgas verließ über Leitung 11, Kühler (W) und Leitung 12 den Reaktor. Der Reaktorinnendruck wurde über einen Druckaufnehmer in Leitung 9 und ein Regelventil in Leitung 12 gehalten.

Mit Hilfe eines Steigrohres wurden über Leitung 3 und das Entspannungsventil (V) stündlich ca. 220 ml Reaktionslösung entnommen, auf ca. 100 mbar entspannt und im Dünnschichtverdampfer (B) bei 80°C entwässert. Verdampftes Phenol/ Wassergemisch wurde über Leitung 6 entfernt und in Tiefkühlfallen (F) (-78°C) vor der Vakuumpumpe (K) kondensiert. In den Kühlfallen (F) fielen stündlich ca. 20 - 30 ml Gemisch an.

Die entwässerte Reaktionslösung gelangte über Leitung 4 die ventillose Pumpe (P) und Leitung 5 wieder in den Reaktor.

Über Leitung 10 wurde mit Hilfe der Pumpe (PP) pro Stunde ca. 500 ml Reaktionslösung entnommen. Dem ausgeschleusten Reaktionsgemisch wurde stündlich eine Probe entnommen und gaschromatographisch analysiert. Nach ca. 4 h war die Apparatur im Gleichgewicht. Die Analysen ergaben, daß in den Reaktionslösungen 12 % Diphenylcarbonat enthalten waren.

## Patentansprüche

1. Verfahren zur Herstellung eines aromatischen Carbonats der Formel
R-O-CO-O-R (I),
in der
R substituiertes oder nicht substituiertes C₆-C₁₂-Aryl bedeutet, durch Umsetzung einer aromatischen Hydroxyverbindung der Formel
R-OH (II),
worin R die oben angegebene Bedeutung hat,
mit Kohlenmonoxid und Sauerstoff in Gegenwart eines Platinmetall-Katalysators, eines Cokatalysators, eines quartären Salzes und einer Base bei einer Temperatur von 30 bis 200°C und einem Druck von 1 bis 200 bar, bei dem ein Teil der Reaktionslösung oder die gesamte Reaktionslösung entspannt wird, bei vermindertem Druck aus der Reaktionslösung Wasser entfernt wird und die entwässerte Reaktionslösung anschließend wieder der Reaktion zurückgeführt wird.

2. Verfahren gemäß Anspruch 1, bei dem kontinuierlich ein Teil der Reaktionslösung entnommen, unter vermindertem Druck entwässert und anschließend der Reaktion wieder zugeführt wird.

3. Verfahren gemäß Anspruch 2, bei dem der pro Stunde entnommene Teilstrom an Reaktionslösung das 0,01 bis 30fache des Reaktorinhalts beträgt.

4. Verfahren gemäß einem der Ansprüche 1 bis 3, bei dem die Wasserentfernung unter weitgehend isothermen Bedingungen durchgeführt wird.

5. Verfahren gemäß Anspruch 4, bei dem die Temperatur bei der Wasserentfernung nicht mehr als 30°C von der Reaktionstemperatur abweicht.

6. Verfahren gemäß einem der Ansprüche 1 bis 5, bei dem die Wasserentfernung bei einem Druck von 1 bis 5000 mbar durchgeführt wird.

## Claims

1. Process for the production of an aromatic carbonate of the formula
R-O-CO-O-R (I)
in which
R signifies substituted or non-substituted C₆-C₁₂ aryl, by reaction of an aromatic hydroxy compound of the formula
R-OH (II)
in which R has the significance specified above,
with carbon monoxide and oxygen in the presence of a platinum-group metal catalyst, a co-catalyst, a quaternary salt and a base at a temperature of 30 to 200°C and a pressure of 1 to 200 bar, wherein a part of the reaction solution or the entire reaction solution is subjected to pressure relief, water is removed from the reaction solution at reduced pressure and the dehydrated reaction solution is subsequently resupplied to the reaction.

2. Process according to Claim 1, wherein a part of the reaction solution is continuously withdrawn, dehydrated under reduced pressure and subsequently resupplied to the reaction.

3. Process according to Claim 2, wherein the partial flow of reaction solution withdrawn per hour amounts to 0.01 to 30 times the content of the reactor.

4. Process according to one of Claims 1 to 3, wherein the removal of water is carried out under largely isothermal conditions.

5. Process according to Claim 4, wherein the temperature in the course of the removal of water differs by no more than 30°C from the reaction temperature.

6. Process according to one of Claims 1 to 5, wherein the removal of water is carried out at a pressure of 1 to 5,000 mbar.

## Revendications

1. Procédé pour préparer un carbonate aromatique de formule
R-O-CO-O-R (I),
dans laquelle
R représente un groupe aryle en C₆-C₁₂ substitué ou non, par réaction d'un composé aromatique hydroxylé de formule
R-O-H (II)
dans laquelle R a les significations indiquées ci-dessus,
avec le monoxyde de carbone et l'oxygène en présence d'un catalyseur à base d'un métal de la série du platine, d'un catalyseur auxiliaire, d'un sel quaternaire et d'une base, à une température de 30 à 200 °C et une pression de 1 à 200 bars, dans lequel on détend une partie de la solution de réaction ou la totalité de la solution de réaction, on en élimine l'eau sous vide et on recycle la solution de réaction déshydratée à la réaction.

2. Procédé selon la revendication 1, dans lequel on prélève en continu une partie de la solution de réaction, on la déshydrate sous vide et on la recycle à la réaction.

3. Procédé selon la revendication 2, dans lequel le courant partiel de la solution de réaction prélevé à l'heure représente de 0,01 à 30 fois le contenu du réacteur.

4. Procédé selon l'une des revendications 1 à 3, dans lequel l'eau est éliminée dans des conditions pratiquement isothermes.

5. Procédé selon la revendication 4, dans lequel la température observée pour l'élimination de l'eau ne s'écarte pas de plus de 30 °C de la température de réaction.

6. Procédé selon l'une des revendications 1 à 5, dans lequel l'eau est éliminée sous une pression de 1 à 5 000 mbars.
